# EUROPEAN PATENT SPECIFICATION

(11) **EP 2 838 518 B1**
(45) Date of publication and mention of the grant of the patent: **21.02.2018**
(21) Application number: 13728512.8
(22) Date of filing: 19.03.2013
(51) Int. Cl.: A61K 9/24, A61K 9/50, A61K 31/65, A61P 17/10

(54) **METHOD OF TREATING ACNE**
VERFAHREN ZUR BEHANDLUNG VON AKNE
PROCÉDÉ DE TRAITEMENT DE L'ACNÉ

(30) Priority: 19.04.2012 US 201261635606 P
(43) Date of publication of application: 25.02.2015
(73) Proprietor: Galderma S.A., 6330 Cham (CH)
(72) Inventor: MANNA, Vasant, Kumar, Ewing, NJ 08638-1724 (US); SEGURA, Sandrine, 06410 Biot (FR); BUSSARD, Ludovic, 73410 Saint-Girod (FR); ETCHEGARAY, Jean-Pierre, 06000 Nice (FR); FREIDENREICH, Philip, Yardley, PA 19067 (US)
(74) Representative: V.O.
(86) International application number: PCT/IB2013/000947
(87) International publication number: WO 2013/156853

(56) References cited:
- WO-A2-2004/091483
- EDWARD MONK ET AL: "Clinical applications of non-antimicrobial tetracyclines in dermatology", PHARMACOLOGICAL RESEARCH, vol. 63, no. 2, 19 October 2010 (2010-10-19), pages 130-145, XP028128784, ISSN: 1043-6618, DOI: 10.1016/J.PHRS.2010.10.007 [retrieved on 2010-10-10]

## Description

### BACKGROUND OF THE INVENTION

Acne is a common skin disease, characterized by areas of skin with seborrhea (scaly red skin), comedones (blackheads and whiteheads), papules (pinheads), pustules (pimples), nodules (large papules) and possibly scarring: Adityan et al., Indian J Dermatol Venereol Leprol 75 (3): 323-6 (2009). Multi-factors contribute to the development acne, such as plugging of the hair follicle with abnormally cohesive desquamated cells, proliferation and colonization of bacteria (e.g., *Propionibacterium acnes),* local inflammation, and abnormalities in follicular keratinization and sebum production. Antibiotics, such as erythromycin, clindamycin, and tetracyclines have been used to treat acne, often severe cases, by topical or oral administration.

Doxycycline is a type of tetracycline broad-spectrum antibiotic that is used to treat a variety of infections and conditions, including prostatitis, sinusitis, syphilis, chlamydia, and pelvic inflammatory disease. Doxycycline is used as a prophylactic antibiotic against malaria and anthrax, as well as for the treatment of diseases such as Lyme disease. Tetracyclines interfere with the protein synthesis of Gram-positive and Gram-negative bacteria by preventing the binding of aminoacyl-tRNA to the ribosome. Their action is bacteriostatic (preventing growth of bacteria) rather than killing (bactericidal). The doses commonly used for doxycycline to achieve antibiotic effects are 100 mg and 50 mg. At relatively high doses, doxycycline has both anti-inflammatory and antimicrobial effects.

Doxycycline, as well as other tetracyclines, also has other therapeutic uses in addition to its antibiotic properties. For example, doxycycline is known to inhibit the activity of collagen destruction enzymes such as collagenase, gelatinase, and elastase. Its collagenase inhibition activity has been used to treat periodontal disease. For another example, doxycycline can inhibit lipase produced by the bacterium *P. acnes* and thus, reduces the availability of free fatty acids that are involved in inflammation. Doxycycline may also reduce inflammation by reducing cytokine levels so that the integrity of the follicular wall is preserved. Thus, at lower doses, doxycycline is also used to treat skin conditions such as rosacea and chronic acne by capitalizing on the anti-inflammatory effects.

Delayed-release or sustained-release technology is often used in tablets and capsules so that they dissolve slowly and release a drug over time. This technology is advantageous because such sustained-release tablets or capsules can be taken less frequently than instant-release preparations and maintains steady levels of the drug in the bloodstream. In a delayed-release tablet or capsule, the Active Pharmaceutical Ingredient (API) may be embedded in a matrix of insoluble substances so that the dissolving drug must find its way out through holes in the matrix. Other drugs are encased in polymer-based tablets with a laser-drilled hole on one side and a porous membrane on the other side. Stomach acids push through the porous membrane, thereby pushing the drug out through the laser-drilled hole. In time, the entire drug dose releases into the system while the polymer container remains intact, to be later excreted through normal digestion. In some sustained release formulations, the drug dissolves into the matrix, and the matrix physically swells to form a gel, allowing the drug to exit through the gel's outer surface.

Oral administration of doxycycline, at a dosage of 50-100 milligrams, once or twice daily, has been used to reduce inflammatory lesions associated with acne. However, these antimicrobial dosages are often associated with the emergence of resistant bacteria and increased frequency and severity of adverse effects, such as nausea, headache, vomiting, etc.

It was reported that twice daily oral administration of sub-antimicrobial dosage (SD) (20 milligrams) doxycycline for 6 months significantly reduced the number of inflammatory and non-inflammatory lesions in patients with moderate facial acne, without causing a detectable antimicrobial effect on the skin flora and an increase in the number or severity of resistant organisms. However, the efficacy was not as pronounced for less than 6 month treatment. For example, at 4 months of treatment, the clinical relevant mean reductions in total inflammatory lesions was 36% for the doxycycline group, while the mean reductions for the placebo was 29%: Skidmore et al., Arch Dermatol. 139:459-464 (2003). The clinical results underlying the foregoing report are described in Monk E. et al., Pharmacological Research, 63(2):130-145 (2010) which also reviews other clinical applications of non-antimicrobial tetracyclines in dermatology, including the clinical testing of sub-antimicrobial doxycycline in the form of 40 mg once daily (controlled release: 30 mg immediate-release; 10 mg delayed-release) for rosacea. WO 2004/091483 describes a pharmaceutical composition of doxycycline that contains an immediate-release (IR) component and a delayed-release (DR) component of the drug which are combined into one dosage units for once-daily dosing. The components can be present in various ratios, with 75:25 IR:DR being preferred, with the total dosage of doxycycline being less than 50 mg, and preferably about 40 mg. A method is also described for treating diseases or conditions in which collagenase is produced in excessive amounts, such as periodontal disease, rheumatoid arthritis and acne, wherein a daily dose of the aforementioned pharmaceutical composition is to be administered.

Compliance with acne treatment regimens, particularly in adolescent patient population, is generally low. The design of the treatment regimen, e.g., frequency and duration of the treatment, the efficacy of the treatment, and the frequency and severity of adverse effects all affect patient compliance. There is an unmet need of an improved treatment for acne that is safe, effective and easy to comply.

### BRIEF SUMMARY OF THE INVENTION

It has been discovered that 16-week, once daily, oral administration of 40 mg doxycycline in a pharmaceutical composition containing 30 mg doxycycline in an immediate release portion and 10 mg doxycycline in a delayed release portion is effective in treating acne. Surprisingly, the 16-week, once daily, oral administration of 40 mg doxycycline has achieved the same or superior efficacy than that of 100 mg doxycycline, with a significant reduction in adverse events.

In one general aspect, the present invention provides a pharmaceutical composition comprising 40 mg doxycycline or a pharmaceutically acceptable salt thereof for use in treating acne in a subject in need of the treatment, wherein the pharmaceutical composition comprises 30 mg doxycycline in an immediate release portion and 10 mg doxycycline in a delayed-release portion and wherein the pharmaceutical composition is to be orally administered to the subject once daily for a treatment period of 16 weeks.

Other aspects, features, and advantages of the invention will be apparent from the following disclosure, including the detailed description of the invention and its preferred embodiments and the appended claims.

### BRIEF DESCRIPTION OF THE SEVERAL VIEWS OF THE DRAWINGS

The foregoing summary, as well as the following detailed description of the invention, will be better understood when read in conjunction with the appended drawing. It should be understood, however, that the invention is not limited to the drawing shown.

In the drawing:

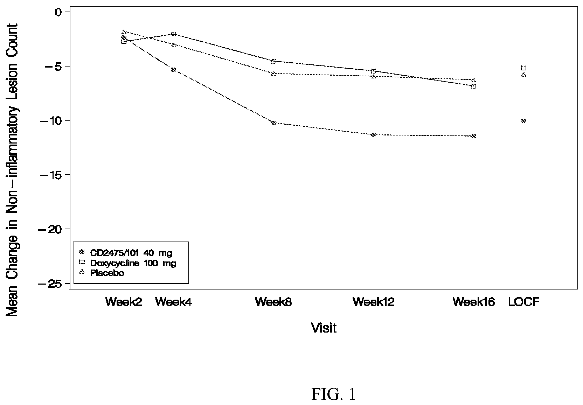

Fig. 1 shows the mean change in non-inflammatory lesion count (ITT Population) from a clinical study.

### DETAILED DESCRIPTION OF THE INVENTION

Unless defined otherwise, all technical and scientific terms used herein have the same meaning as commonly understood to one of ordinary skill in the art to which this invention pertains. Otherwise, certain terms used herein have the meanings as set in the specification. It must be noted that as used herein and in the appended claims, the singular forms "a," "an," and "the" include plural reference unless the context clearly dictates otherwise.

Embodiments of the present invention relate to a pharmaceutical composition comprising 40 mg doxycycline or its pharmaceutically acceptable salt for use in treating acne in a subject in need of the treatment, wherein the 40 mg of doxycycline consists of 30 mg immediate release portion and 10 mg of delayed-release portion and wherein the pharmaceutical composition is to be orally administered to the subject once daily for 16 weeks.

According to an embodiment of the present invention, the so-described treatment results in reduction of inflammatory lesion count in the subject.

According to another embodiment of the present invention, the so-described treatment results in reduction of non-inflammatory lesion count in the subject.

In yet another embodiment of the present invention, the treatment results in increased success rate in the subject.

As used herein, "success rate" means the percentage of subjects who achieve a score of "clear" or "almost clear" by Investigator's Global Assessment (IGA) (Inflammatory):

| **Score** | **Grade** | **Description** |
|---|---|---|
| 0 | Clear | No evidence of papules or pustules (inflammatory lesions) |
| 1 | Almost Clear | Rare non-inflamed papules (papules must be resolving and may be hyperpigmented, though not pink-red) |
| 2 | Mild | Few inflammatory lesions (papules/pustules only; no nodulocystic lesions) |
| 3 | Moderate | Multiple inflammatory lesions evident: many papules/pustules; up to two nodulocystic lesions |
| 4 | Severe | Inflammatory lesions are more apparent, many papules/pustules, may be a few nodulocystic lesions |

According to an embodiment of the present invention, a pharmaceutical composition useful for the present invention comprises 30 mg immediate release dose of doxycycline, and 10 mg delayed-release dose of doxycycline in a unit dosage. The pharmaceutical composition can be in a tablet or a capsule form.

The immediate release portion of the composition can be in the form of powder, granule, beadlet, or tablet; the delayed-release portion can be coated granular, coated beadlet, coated tablet, or uncoated matrix tablet. Several dosage form variations can be used to achieve a product with these attributes. For example, an immediate-release powder blend can be encapsulated with a delayed-release tablet or delayed-release pellets. A further example is an immediate-release tablet and a delayed-release tablet that are prepared separately and encapsulated into an appropriate sized capsule shell. Or, for example, a delayed-release tablet can be used as a core and the immediate-release portion can be compressed as an outer layer using a press coater or overcoated using a drug layering technique, both techniques of which can be found in Gunsel and Dusel, Chapter 5, "Compression-coated and layer tablets", in Pharmaceutical Dosage Forms: Tablets, Second Edition, Volume 1, Edited by H. A. Lieberman, L. Lachman, and J. B. Schwartz, Marcel Dekker, Inc. New York and Basel (1990).

Described herein is a method for making a sustained-release tablet that comprises a core tablet containing a delayed release (DR) portion of active ingredient and four coating layers: an inner subcoating layer, an enteric coating layer, an active layer containing an immediate release (IR) portion of active ingredient, and an outer coating layer. Such a formulation allows for both immediate and delayed release of the active ingredient. In an embodiment of the present invention, the core tablet of such a sustained-release tablet contains 10 mg of the chemically modified tetracycline (CMT), doxycycline, as the API, and the active layer contains 30 mg of the API. The resulting sustained-release tablet thus provides 40 mg of API in a ratio of IR:DR of about 3:1.

A method for preparing such sustained-release tablets comprises:
(a) wet granulating a first portion of an active ingredient and at least one inactive ingredient to produce a wet granulate;
(b) drying the wet granulate;
(c) milling the dried granulate;
(d) blending the milled granulate with at least one external phase;
(e) compressing the blend to form a core tablet;
(f) coating the core tablet with at least one inner coating layer comprising at least a first polymer and a first plasticizer;
(g) coating the inner layer-coated tablet with at least one enteric coating layer comprising at least one enteric material;
(h) coating the enteric layer-coated tablet with at least one active layer comprising a second portion of the at least one active ingredient, a second polymer, and a second plasticizer; and
(i) coating the active layer-coated tablet with at least one outer coating layer comprising at least a third polymer and a third plasticizer.

Each method step will be described in more detail below. The critical step for providing a sustained-release tablet with long-term storage stability and content uniformity is step (h), in which the tablet is coated with an active layer containing the API. This step involves preparing a homogeneous suspension of the active material, polymer, and plasticizer (inactive ingredients) and spraying the suspension onto the enteric layer-coated tablet from step (g) using a slow, long spray routine, such as about twenty hours. It is critical when performing step (h) that the application parameters, such as mixture homogeneity, mixing time, and coating time, be closely monitored and controlled to ensure the stability and uniformity of the final sustained release tablet. Further, it is necessary to continuously mix the homogeneous suspension during the spray routine.

### Granulation

The first step in the method involves wet granulating an active ingredient and at least one inactive ingredient to produce a wet granulate. Preferably, the granulating is performed in a high shear wet granulator, such as with a Fielder PMA 100 Granulator or similar instrument. In high shear wet granulation, liquid, such as purified water, is sprayed onto a powder bed concurrent with high shear mixing. The mixing is accomplished via an impeller blade and chopped blade set to pre-determined speeds. The amount of granulating liquid and the speed at which it is sprayed play an important role in the overall quality of the final wet mass and the optimal parameters may be determined by routine experimentation. It has been found effective to utilize a spray rate of about 5-7 liters/minute and about 8-10 liters of liquid, preferably about 9 liters of liquid, for a powder portion of about 20 kg.

The active ingredient included in the granulate is preferably an API, such as a chemically-modified tetracycline. In accordance with an embodiment of the composition for use according to the present invention, the active ingredient is doxycycline. Typically, doxycycline in monohydrate form, which is the base molecule hydrated with one molecule of water, is utilized for forming the granulate. It is also within the scope of the invention to utilize more than one active ingredient. For example, doxycycline may be combined with another therapeutic substance.

Appropriate inactive ingredients for inclusion in the core tablet are well-known in the art, and may include bulking agents, including microcrystalline cellulose, such as AVICEL® (FMC Corp.) or EMCOCEL® (Mendell Inc.); dicalcium phosphate, such as EMCOMPRESS® (Mendell Inc.); calcium sulfate, such as COMPACTROL® (Mendell Inc.); and starches, such as STARCH 1500. Additionally, disintegrating agents, such as microcrystalline cellulose, starches, crospovidone, such as POLYPLASDONE XL® (International Specialty Products); sodium starch glycolate, such as EXPLOTAB® (Mendell Inc.); and croscarmellose sodium (carboxymethyl cellulose sodium), such as AC-DI-SOL® (FMC Corp.), may be utilized as inactive ingredients.

In a preferred embodiment, the inactive ingredients in the core tablet include pregelatinized starch, microcrystalline cellulose, and croscarmellose sodium. Preferably, 10 mg of pure API are combined with about 53 mg of microcrystalline cellulose, about 20 mg starch, and about 2 mg croscarmellose sodium. If the purity of the API is less than 100%, a larger amount of API is preferably included in the core tablet to achieve the desired amount of active ingredient. In such case, the amount of microcrystalline cellulose is preferably decreased to maintain the same total weight in the wet granulate.

### Drying

Following granulation, the wet granulate is dried, such as with a fluid bed dryer in a preferred embodiment. An O'Hara Fluid Bed Dryer or similar instrument would be appropriate. Fluid bed drying is a process in which hot air is fed through a wet mass at a velocity such that the powders behave like a fountain. This imparts a drying effect on the wet mass that can be quantitatively measured using a "loss on drying" instrument. Parameters that can be adjusted include inlet air temperature and inlet air volume. If the temperature is too low, the powders will take longer to dry, which negatively affects efficiency. On the other hand, if the temperature is too high, case hardening can occur, in which the outer layer of the granule becomes hard and does not allow the inner layer of the granule to dry properly. Inlet air volume is also an important parameter. If the air volume is too low, the powders will not fluidize properly and uneven drying will occur. If the air volume is too high, product will be blown into the filters and the yield of the batch will be compromised. It is noted that air volume is a parameter that is rarely constant during the duration of the run. Rather, air volume must be closely monitored during processing and adjustments may be necessary. At the beginning of a run, the volume is typically set to a higher level in order to get the heavier wet mass fluidizing. As the powders dry, air volume is lowered to protect against losing the powders in the filters. A preferred drying temperature is about 60 to 65°C.

### Milling

After drying, the dried granulate (powder) is milled, such as by using a Fitzmill in a preferred embodiment. Milling is a high-energy operation in which large granules are re-sized in order to be better suited for blending and tablet compression. Milling also tightens particle size distribution to enhance uniformity and mitigate powder separation. Particle size plays an important role in powder flow characteristics and compressibility. Many times, flow and compressibility are inversely proportional to each other, so finding an optimal balance is vital.

Milling of the product may be accomplished in two ways: "knives forward" and "impacts forward." Knives forward works to break up large, hard granules, whereas impacts forward is good for pulverizing smaller particle size ingredients, such as active ingredients. In the method of the invention, milling is preferably performed using knives forward because a higher-energy, cutting motion is needed to break up the granules.

The screen size which provides the best results must be determined by routine experimentation. When doxycycline is used as the API in combination with pregelatinized starch, microcrystalline cellulose, and croscarmellose sodium, a preferred screen size is about 838 µm (0.033").

### Blending

Following milling, the granulate is blended with an external phase, including glidents and lubricants, to form a homogeneous mixture and enhance uniformity. Blending may be performed using a low shear cubic tumble blender, for example. The blending may be accomplished in one or more stages. For example, in a three-stage blending process, intragranular material is mixed first, followed by the addition of secondary ingredients such as glidents, followed by lubricant(s). Good blend uniformity is very important as it can directly correlate with content uniformity. In a single- or multi-stage blending process, blending at each stage must be performed for a sufficient time that a homogeneous blend is produced. The appropriate blending time may be easily determined by routine experimentation.

In a preferred embodiment, AC-DI-SOL® (croscarmellose sodium) and AVICEL® PH 200 (microcrystalline cellulose) are utilized as glidents and magnesium stearate is included as a lubricant. A three-stage blending process is presently preferred. Specifically, the intragranular portions (containing 10 mg doxycycline, about 53 mg microcrystalline cellulose, about 20 mg starch, and about 2 mg croscarmellose sodium) are mixed for about ten minutes, blended with about 12.5 mg microcrystalline cellulose and 2 mg croscarmellose sodium for about 30 minutes, then blended with about 0.5 mg magnesium stearate for about 6 to 8 minutes, preferably about 7 minutes. It is possible to use greater or lesser amounts of the lubricants and glidents, such as may be determined by routine experimentation.

### Compressing

Finally, the mixture is compressed into a tablet. For example, in a typical compression (tableting) step, the blend is fed into a feed frame which is located at the top of a press die table. The feed frame moves the blend around and into a die, which then travels around the press and is compressed when the lower and upper punch come together. The lower punch then rises to eject the tablets through an ejection chute. A Manesty unipress or similar instrument would be appropriate for the compressing step.

It is necessary to determine acceptable upper and lower limits for tablet physical characteristics, such as tablet hardness, disintegration time, and gauge thickness, as well as tablet weight and friability. Additionally, defects in tablets are not desirable. Variables such as feed frame speed and compression force may be varied to achieve the desired tablet properties. Preferably, tablet press speed is about 2000 to 2600 tablets per minute and feed frame speed is about 8-12 rpm.

The resulting tablet may now be considered a "core tablet" containing a delayed release portion of API. Preferably, a 100 mg core contains 10% API, about 65.5 % microcrystalline cellulose, about 20% pregelatinized starch, about 4% croscarmellose sodium, and about 0.5% magnesium stearate.

### Coating Layers

After compression, the resulting core tablets are coated with multiple coating layers. Preferably, the tablets are sequentially coated with four coating layers: an inner (subcoating) layer, an enteric coating layer, an active layer containing the immediate release portion of API, and an outer (overcoating) layer. The resulting coated sustained-release tablet thus allows for both immediate and delayed release of the API. Preferably, the four coating layers for a 100 mg core tablet have a total weight of about 82 mg, resulting in a sustained-release tablet having a total weight of about 182 mg. The core tablet thus comprises about 55 wt% of the weight of the sustained-release tablet and the coating layers comprise about 45 wt %.

### Inner Coating Layer

The inner coating layer comprises at least a first polymer and a first plasticizer. Preferably, the inner coating layer or subcoating contains a polymer, plasticizer, and pigment, such as a subcoating which comprises 10% OPADRY® (Colorcon, Inc., USA) in water. For example, the coating may be prepared by dissolving the OPADRY® in purified water to 10% solids content and mixing until completely dissolved, such as for at least about 45 minutes. If foam is present after mixing, the mixture may be allowed to settle, such as for at least one hour. The purpose of the inner layer is to protect the core tablet from the acidic enteric coating that will be subsequently applied. The appropriate amount of subcoating, which may be evaluated or assessed by weight gain per tablet, may be determined by routine experimentation. For example, for a 100 mg core tablet, about 3 to 5 mg, preferably about 4 mg, of OPADRY® has been found to be effective (representing about 2% of the total weight of the 182 mg sustained-release tablet). A particularly preferred inner layer comprises OPADRY® 03 K 19229 (Colorcon, Inc.), which contains hypromellose 6cP, triacetin, and talc. However, other similar coating compositions that are known in the art or to be developed would also be suitable.

### Enteric Coating Layer

An enteric coating comprising at least one enteric material is applied to the inner layer-coated tablet to delay the release of the API from the tablet. Enteric materials are polymers that are substantially insoluble in the acidic environment of the stomach, but are predominantly soluble in intestinal fluids at specific pHs. The enteric coating thus delays the release of the API from the core tablet, allowing the tablet to pass through the stomach intact and release the API in the intestine.

Appropriate enteric materials are non-toxic, pharmaceutically acceptable polymers, and include, without limitation, cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and commercially available enteric dispersion systems.

Preferably, the enteric coating comprises ACRYL-EZE® (Colorcon, Inc., USA), an aqueous acrylic enteric system comprising methacrylic acid co-polymer type C. To form the enteric coating, solid ACRYL-EZE®, for example, may be dispersed in purified water to a 10% solids content by mixing until a homogeneous dispersion is achieved. Preferably, the solid is gradually added to water (such as over at least about 45 minutes) with mixing to minimize amounts of large polymer formation, which may clog spray nozzles. Screening, such as through a 595 µm (30 mesh) screen, may be necessary prior to spraying of the coating on the tablet. A particularly preferred enteric material is ACRYL-EZE® 93 F 19255 (Colorcon, Inc.) which contains, in addition to methacrylic acid copolymer type C, talc, Macrogol (PEG 8000), colloidal anhydrous silica, sodium bicarbonate, and sodium lauryl sulfate.

The appropriate amount of enteric coating needed to protect the core tablet from acidic media may be evaluated or assessed by weight gain per tablet and may be determined by routine experimentation. For a 100 mg core tablet, a layer of about 10 to about 15 mg, preferably about 12.5 mg, of enteric coating has been found to be particularly effective. This represents about 7 wt% of the total weight (182 mg) of the sustained-release tablet.

### Active Layer

The third coating layer is an active layer. This layer contains the immediate release portion of the API and an inactive ingredient comprising at least a polymer and a plasticizer, and is critical to providing the sustained-release tablet with the necessary stability, including content uniformity. In one embodiment, the polymer and plasticizer contained in the active layer are the same as those contained in the inner coating layer.

An appropriate active layer contains OPADRY®, API, and water, and has about 15% solids content. It has been found effective to utilize a ratio of API to inactive ingredient of about 1:1 (50 wt% active ingredients), such as about 28.5 mg OPADRY® and about 30 mg of API to yield an active layer weighing about 58 mg. The amount of API may vary according to its purity through a corrective factor. In accordance with an embodiment of the composition for use according to the present invention, a 100 mg core tablet containing 10 mg API is coated with an active layer containing 30 mg API, yielding a sustained-release tablet containing 40 mg API (about 22 wt% of a 182 mg sustained release tablet) in a ratio of IR to DR of 3:1, wherein the API is doxycycline.

The active layer is preferably prepared by dispersing the active and inactive ingredient in water and mixing for at least 45 minutes, preferably at least about 60 minutes, to achieve a homogeneous suspension. Alternatively, the inactive and active ingredients may be added sequentially to water and mixed for at least one hour following each addition. It is very important that the suspension be mixed until complete dispersion of the API and inactive ingredients is achieved. The suspension must also be mixed continuously during coating to achieve uniformity, which is particularly critical for this coating layer because it contains active ingredient. Coating time is also important, and is preferably about 20 hours for coating this layer.

### Outer Layer

The outer coating layer or overcoating provides protection for the sustained-release tablet and contains a polymer and plasticizer. The polymer and plasticizer may be the same or different as those contained in the inner and active coating layers. For example, the overcoating layer may be identical to the inner coating layer, such as a layer containing 10% OPADRY® in water. It has been found effective to utilize an outer coating layer representing about 3 to 5% by weight, preferably about 4% by weight of the total weight of the sustained release tablet, such as about 7 mg coating for a 100 mg core tablet.

### Application of Coating Layers

Each of the four coating layers is applied by spraying, such as with an O'Hara fastcoat device with a peristaltic pump. Following preparation of the appropriate coating solution or suspension, the tablets are placed in a coating pan and heated, such as for about ten minutes, before coating spray begins. During coating, intermittent weight checks of tablets may be performed to track weight gain. Once the target weight gain for each coating has been achieved, the tablets are cooled to room temperature. They are then ready for packaging.

Variables in coating include number of spray guns, distance between spray guns, gun distance from tablet bed, and spray nozzle size. The distance between spray guns is important to ensure uniform spray across the entirety of the tablet bed. Additionally, the distance between the spray guns and the tablet bed needs to be kept relatively constant to ensure a good spray pattern and results. As the coating continues and the tablet grows in size, the spray gun manifold will need to be moved back to maintain an appropriate distance. The appropriate size of the nozzle may be determined based on the particle size in the suspension of the particular coating suspension. It has been found effective to utilize a 724 µm (0.0285") diameter spray nozzle for application of the inner, active, and outer coatings, and a 0.15 cm (0.060") diameter spray nozzle for application of the enteric coating.

Spray rate, pan speed, and air temperature are critical for applying the four coating layers in order to produce sustained release tablets with the desired stability profiles. Preferred parameters include spray rates of about 350 to 400 ml/min, pan speed of about 5 - 8 rpm, and air temperature of about 50 to 60 °C. It has been found effective to utilize a coating time of about 4-6 hours for coating the inner, enteric, and outer layers. However, for coating the active layer, spraying at a rate of about 375 to 400 ml/min with a pan speed of about 7 to 9 rpm and for a total spray time of about 20 hours is important for providing the desired API layer stability.

It is believed that the stability of the sustained release tablets described herein above is due to the natural API stability and the presence of the four coating layers: an inner layer, an enteric layer, an active layer, and an outer protective layer. Additionally, the observed content uniformity is due to a combination of the core tablet content and the API layer content. It is thus critical to carefully control the uniformity of the API layer.

Sustained-release tablets prepared according to the method described herein were found to exhibit improved properties relative to prior art tablets when evaluated for appearance and dissolution after three months storage. Similar results are expected for longer storage times.

As previously explained, the API for inclusion in the sustained release tablets according to an embodiment of the invention is doxycycline. A method of making a sustained-release doxycycline tablet comprises:
(a) wet granulating about 10 to 12% wt% doxycycline and about 88-90% of at least one bulking or binding agent to produce a wet granulate;
(b) drying the wet granulate;
(c) milling the dried granulate;
(d) blending the milled granulate with at least one glident and at least one lubricant to form a blend,
(e) compressing the blend to form a core tablet comprising about 55 wt% of the weight of the sustained-release tabled;
(f) coating the core tablet with at least one inner coating layer comprising a first polymer and a first plasticizer, wherein the inner layer comprises about 2 wt% of the weight of the sustained-release tablet;
(g) coating the inner layer-coated tablet with an enteric coating layer comprising at least one enteric material, wherein the enteric coating layer comprises about 7 wt % of the weight of the sustained-release tablet;
(h) coating the enteric layer-coated tablet with at least one active layer comprising doxycycline and a coating material comprising a second polymer and a second plasticizer, wherein a ratio of doxycycline to coating material is about 1:1, and wherein the doxycycline comprises about 22% of the weight of the sustained release tablet; and
(i) coating the active layer-coated tablet with at least one outer coating layer comprising at least a third polymer and a third plasticizer, wherein the outer layer comprises about 4 wt% of the sustained release tablet.

Step (h) involves preparing a homogeneous suspension comprising the doxycycline, second polymer, and second plasticizer by mixing for about one hour, and spraying the homogeneous suspension onto the enteric layer-coated tablet for about twenty hours. Importantly, the homogeneous suspension is mixed continuously during the spraying. Each of the steps in the method of making a sustained release doxycycline tablet has been previously described in detail herein above.

### Multiparticulate Capsules

As a preferred embodiment, the IR/DR composition of doxycycline is in the form of a capsule containing beadlets. At present, it is preferred to have two different types of units in a single form multiple-unit dosage form.

The first unit is an immediate release dosage form, preferably in pellet form. This component can also be a powder if desired or necessary. In either case, the dosage form may have a surface-active agent such as sodium lauryl sulfate, sodium monoglycerate, sorbitan monooleate, polyoxyethylene sorbitan monooleate, glyceryl monostearate, glyceryl monooleate, glyceryl monobutyrate, any one of the Pluronic line of surface-active polymers, or any other suitable material with surface active properties or any combination of the above. Preferably, the surface-active agent would be a combination of sodium monoglycerate and sodium lauryl sulfate. The concentration of these materials in this component can range from about 0.05 to about 10.0% (w/w).

Other excipient materials that can be employed in making drug-containing pellets are any of those commonly used in pharmaceutics and should be selected on the basis of compatibility with the active drug and the physicochemical properties of the pellets. These include, for instance: binders such as cellulose derivatives such as methylcellulose, hydroxyethyl cellulose, hydroxypropyl cellulose, hydroxypropyl methylcellulose, polyvinylpyrrolidone, polyvinylpyrrolidone/vinyl acetate copolymer and the like; disintegration agents such as cornstarch, pregelatinized starch, cross-linked carboxymethylcellulose (AC-DI-SOL®), sodium starch glycolate (EXPLOTAB®), cross-linked polyvinylpyrrolidone (PLASDONE® XL), and any disintegration agents used in tablet preparations, which are generally employed in immediate release dosages such as the one underlying the present invention; filling agents such as lactose, calcium carbonate, calcium phosphate, calcium sulfate, microcrystalline cellulose, dextran, starches, sucrose, xylitol, lactitol, mannitol, sorbitol, sodium chloride, and polyethylene glycol; surfactants such as sodium lauryl sulfate, sorbitan monooleate, polyoxyethylene sorbitan monooleate, bile salts, glyceryl monostearate, and the PLURONIC® line (BASF); solubilizers such as citric acid, succinic acid, fumaric acid, malic acid, tartaric acid, maleic acid, glutaric acid sodium bicarbonate and sodium carbonate; and stabilizers such as any antioxidation agents, buffers, and acids can also be utilized.

The pellet can be made by, for example, simple granulation, followed by sieving; extrusion and marumerization; rotogranulation; or any agglomeration process that results in a pellet of reasonable size and robustness. For extrusion and marumerization, the drug and other additives are granulated by addition of a binder solution. The wet mass is passed through an extruder equipped with a certain size screen, and the extrudates are spheronized in a marumerizer. The resulting pellets are dried and sieved for further applications. One may also use high-shear granulation, wherein the drug and other additives are dry-mixed and then the mixture is wetted by addition of a binder solution in a high shear-granulator/mixer. The granules are kneaded after wetting by the combined actions of mixing and milling. The resulting granules or pellets are dried and sieved for further applications.

As stated earlier, it is also possible to have this immediate release component as a powder, although the preferred form is a pellet due to mixing and de-mixing considerations.

Alternatively, the immediate release beadlets or pellets of the composition can be prepared by solution or suspension layering, whereby a drug solution or dispersion, with or without a binder, is sprayed onto a core or starting seed (either prepared or a commercially available product) in a fluid bed processor or other suitable equipment. The cores or starting seeds can be, for example, sugar spheres or spheres made from microcrystalline cellulose. The drug thus is coated on the surface of the starting seeds. The drug-loaded pellets are dried for further applications.

The second unit should have a delayed release (DR) profile, and needs to be able to address the changing pH of the GI tract, and its effect on the absorption of doxycycline. This pellet should have all of the ingredients as mentioned for the first unit pellet, as well as optionally some organic acid that will be useful to reduce the pH of the microenvironment of the pellet, and thus facilitate dissolution. These materials are, but not limited to, citric acid, lactic acid, tartaric acid, or other suitable organic acids. These materials should be present in concentrations of from about 0 to about 15.0% (w/w); preferably these materials would be present in concentrations of from about 5.0 to about 10.0 percent (w/w). The process for manufacturing these pellets is consistent with the process described above for the first unit pellet.

Unlike the first unit pellet, the second unit delayed-release component has a controlling coat applied to the surface of the pellet such that the release of the drug from the pellet is delayed. This is accomplished by applying a coating of enteric materials. "Enteric materials" are polymers that are substantially insoluble in the acidic environment of the stomach, but are predominantly soluble in intestinal fluids at specific pHs. The enteric materials are non-toxic, pharmaceutically acceptable polymers, and include, for example, cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (e.g., EUDRAGIT® L30D55, EUDRAGIT® FS30D, EUDRAGIT® L100, KOLLICOAT® EMM30D, ESTACRYL® 30D, COATERIC®, and AQUATERIC®). The foregoing is a list of possible materials, but one of skill in the art would recognize that it is not comprehensive and that there are other enteric materials that would meet the objectives of providing for a delayed release profile. These coating materials can be employed in coating the surfaces in a range of from about 1.0% (w/w) to about 50% (w/w) of the pellet composition. Preferably, these coating materials should be in a range of from about 20 to about 40 percent (w/w). The pellets may be coated in a fluidized bed apparatus or pan coating, for example.

With the enteric coated pellets, there is no substantial release of doxycycline in the acidic stomach environment of approximately below pH 4.5. The doxycycline becomes available when the pH-sensitive layer dissolves at the greater pH of the small intestine; after a certain delayed time; or after the unit passes through the stomach. The preferred delay time is in the range of two to six hours.

As a variation of this embodiment, the DR pellet contains layers of the doxycycline, separated by protective layers, and finally an enteric coating, resulting in a "repeat-action" dosage delivery. Such a dosage form may meet the blood level requirements of the release profile if the release of the doxycycline in all of the layers is within the absorption window for the drug.

An overcoating layer can further optionally be applied to the IR/DR pellets. OPADRY®, OPADRY II® (Colorcon) and corresponding color and colorless grades from Colorcon can be used to protect the pellets from being tacky and provide colors to the product. The suggested levels of protective or color coating are from 1 to 6%, preferably 2-3% (w/w).

Many ingredients can be incorporated into the overcoating formula, for example to improve the coating process and product attributes, such as plasticizers: acetyltriethyl citrate, triethyl citrate, acetyltributyl citrate, dibutylsebacate, triacetin, polyethylene glycols, propylene glycol and others; lubricants: talc, colloidal silica dioxide, magnesium stearate, calcium stearate, titanium dioxide, and magnesium silicate.

The delayed release and immediate release units are combined in the dosage form (in this instance, the different pellets are put into capsules) in a predetermined ratio, preferably about 70:30 to about 80:20, most preferably 75:25 (IR/DR), which will achieve the desired steady state blood serum levels with only once-daily dosing.

The composition, preferably in beadlet form, can be incorporated into hard gelatin capsules, either with additional excipients, or alone. Typical excipients to be added to a capsule formulation include, but are not limited to: fillers such as microcrystalline cellulose, soy polysaccharides, calcium phosphate dihydrate, calcium sulfate, lactose, sucrose, sorbitol, or any other inert filler. In addition, there can be flow aids such as fumed silicon dioxide, silica gel, magnesium stearate, calcium stearate or any other material imparting flow to powders. A lubricant can further be added if necessary by using polyethylene glycol, leucine, glyceryl behenate, magnesium stearate or calcium stearate.

The composition may also be incorporated into a tablet, in particular by incorporation into a tablet matrix, which rapidly disperses the particles after ingestion. In order to incorporate these particles into such a tablet, a filler/binder must be added to a table that can accept the particles, but will not allow their destruction during the tableting process. Materials that are suitable for this purpose include, but are not limited to, microcrystalline cellulose (AVICEL®), soy polysaccharide (EMCOSOY®), pre-gelatinized starches (STARCH® 1500, NATIONAL® 1551), and polyethylene glycols (CARBOWAX®). The materials should be present in the range of 5-75% (w/w), with a preferred range of 25-50% (w/w).

In addition, disintegrants are added in order to disperse the beads once the tablet is ingested. Suitable disintegrants include, but are not limited to: cross-linked sodium carboxymethyl cellulose (AC-DI-SOL®), sodium starch glycolate (EXPLOTAB®, PRIMOJEL®), and cross-linked polyvinylpolypyrrolidone (Plasone-XL). These materials should be present in the rate of 3-15% (w/w), with a preferred range of 5-10% (w/w).

Lubricants are also added to assure proper tableting, and these can include, but are not limited to: magnesium stearate, calcium stearate, stearic acid, polyethylene glycol, leucine, glyceryl behenate, and hydrogenated vegetable oil. These lubricants should be present in amounts from 0.1-10% (w/w), with a preferred range of 0.3-3.0% (w/w).

Tablets are formed, for example, as follows. The particles are introduced into a blender along with AVICEL®, disintegrants and lubricant, mixed for a set number of minutes to provide a homogeneous blend which is then put in the hopper of a tablet press with which tablets are compressed. The compression force used is adequate to form a tablet; however, not sufficient to fracture the beads or coatings.

It will be appreciated that the multiple dosage forms can deliver dosages of pharmaceutically active doxycycline to achieve the desired levels of the drug in a recipient over the course of about 24 hours at steady state with a single daily oral administration.

Further described herein is a method for treating a mammal with doxycycline. The method involves administering a doxycycline composition as described herein to a mammal, preferably a human, in need of the anti-collagenase or anti-inflammatory activity of doxycycline substantially without accompanying antibiotic activity. Systemic administration is preferred, and oral administration is most preferred.

Using the compositions as described herein, the steady state blood levels of doxycycline or other tetracycline of a minimum of about 0.1 µg/ml, preferably about 0.3 µg/ml and a maximum of about 1.0 µg/ml, more preferably about 0.8 µg/ml, can be achieved to treat diseases with increased collagenase production, such as periodontal disease, skin diseases, as well as inflammatory states. Indeed, any disease state treatable with sub-antimicrobial blood levels of a tetracycline given in multiple daily dosages can also be treated using the corresponding once-daily formulations underlying the present invention.

This invention will be better understood by reference to the examples that follow, but those skilled in the art will readily appreciate that the examples are only illustrative of the invention as defined in the claims which follow thereafter.

### Example 1: Preparation of a Tablet Composition

### Wet Granulation

Doxycycline H₂O (10 mg), microcrystalline cellulose (AVICEL® PH 102, 53 mg), pregelatinized starch (20 mg), and croscarmellose sodium (AC-DI-SOL®, 2 mg) were combined in a high shear weight granulator (Fielder PMA 100) and the impact of water spray parameters on wet granulates was evaluated. Variables included blade speed (low or high), spray volume, spray rate (low, medium, and high), and total spray amount. The effects of these variables were assessed by visual observation, loss on drying, and yield. It was concluded that, upon scale-up, a spray volume of about 9 liters for a 25 kg solid portion and a spray rate of about 5 to 7 liters per minute would be optimal.

### Drying

In order to evaluate the impact of drying parameters on dry granulates, the wet granulates from Example 1 were placed in an O'Hara fluid bed dryer and hot air was sprayed on the wet mass. Parameters included temperatures, air volume, and LOD (loss on drying) challenge, which were assessed by visual observation, LOD, and yield. It was concluded that the optimal supply temperature was about 60°C.

### Milling

The dried granulate from Example 2 was milled in a Fitzmill model M using knives forward mode in order to evaluate the impact of screen size on powder flowability. Three screen sizes, namely 559 µm, 838 µm, and 0.17 cm (0.022", 0.033", and 0.065") were studied. The effects of these variables were assessed by visual observations, particle size, bulk and tap density, and yield. It was concluded that the optimal screen size was 838 µm (0.033").

### Blending

The milled granulate from Example 3 was blended in a three-stage blending process using a 0.43 m³ (15 cubic foot) tote blender and a Vortiv-Siv sifter. Intragranular portions (87 mg) were blended in a first step, followed by glidents (2.5 mg AC-DI-SOL® (croscarmellose sodium) and 12.5 mg AVICEL® PH 200 (microcrystalline cellulose), and then lubricant (0.5 mg magnesium stearate). In order to evaluate the homogeneity of granulated powder after introduction of glidents and lubricants, the following parameters were varied: blending time after initial charge of granulated powder, blending time after addition of glidents, and blending time after addition of lubricants. The effects of the blending times were assessed by visual observation and uniformity. It was concluded that the optimal blending time for intragranulated portions was about 10 minutes, after addition of glidents about 30 minutes, and after addition of lubricants about 7 minutes.

### Compressing

This example illustrates the compressing (tableting) step.

In order to evaluate the effect of compression parameters on tablet uniformity, the granulated powder from Example 4 was formed into tablets using a rotary tablet press (Manesty Unipress). Variables included tablet speed, compression force (19, 13, and 7.5 kN and feed frame speed). The effects of these variables were assed by visual observation, hardness, friability, disintegration, thickness, weight, and yield. It was concluded that a tablet press speed of 2000-2600 tablets per minute and a feed frame speed of 8-12 rpm were particularly effective.

### Coating

Four coating layers were sequentially applied. A first (inner) layer was prepared by dissolving OPADRY® in water to a 10% solids content, mixing for at least 45 minutes, and settling for at least 1 hour if foam was present. The coating was applied to the core tablet from Example 5 using an O'Hara fastcoat [1.2 m (48") pan] with a peristaltic pump and three spray guns with a 724 µm (0.0285") diameter nozzle. It was found that a supply temperature of 60°C, a pan speed of 7.0 rpm, and a spray rate of 275-325 ml/minute were particularly effective. The total spraying time was about 4 hours.

An enteric coating layer was prepared by dissolving ACRYL-EZE® in water to a solids content of about 20% by adding the solid to water at a rate of 300 grams per minute, mixing for at least 45 minutes, and controlled by screening through a 595 µm (30 mesh) screen. The coating was applied to the inner layer-coated tablet using an O'Hara fastcoat [1.2 m (48") pan] with a peristaltic pump and three spray guns with a 0.15 cm (0.060") diameter nozzle. It was found that a supply temperature of 50-55°C, a pan speed of 7.0 rpm, and a spray rate of 250-300 ml/minute were particularly effective. The total spraying time was about 5.5 hours.

An active coating layer was prepared by first dissolving OPADRY® in water and mixing for at least one hour, then adding doxycycline and mixing for an additional hour. It was critical to ensure the uniformity of the resulting suspension. The coating was applied to the enteric layer-coated tablet using an O'Hara fastcoat [1.2 m (48") pan] with a peristaltic pump and three spray guns with a 724 µm (0.0285") diameter nozzle. It was found that a supply temperature of 60°C, a pan speed of 7.0-9.0 rpm, and a spray rate of 375-400 ml/minute were particularly effective. The spraying was performed over a long period (about 18.5 hours), and the suspension was mixed continuously during the spraying operation.

Finally, an outer coating layer was prepared by dissolving OPADRY® in water to a 10% solids content, mixing for at least 45 minutes, and settling for at least 1 hour if foam was present. The coating was applied to the tablet from Example 5 using an O'Hara fastcoat [1.2 m (48") pan] with a peristaltic pump and three spray guns with a 724 µm (0.0285") diameter nozzle. It was found that a supply temperature of 60°C, a pan speed of 7.0 rpm, and a spray rate of 275-325 ml/minute were particularly effective. The total spraying time was about 4 hours.

### Example 2: Stability Studies of the Tablet Composition

The stability of sustained-release doxycycline tablets prepared as described above was evaluated by storing the tablets for three months under different temperature and relative humidity conditions and performing periodic evaluations of the tablets in terms of appearance, dissolution profile, stability, impurity profile, and moisture content. One bottle (Bottle A) of 1000 tablets was stored for three months at 25°C and 60% RH and a second bottle of 1000 tablets (Bottle B) was stored for three months at 40°C and 75% RH.

After one, two, and three months, tablets were removed from the bottles for analysis. The tablets were initially round and beige with a single black dot. No change in appearance was observed after one, two, or three months for the tablets in Bottle A or Bottle B at 25°C/60% RH and at 40°C/75% RH, respectively. Furthermore, there were no significant changes in stability, water content, and impurity assay results. Thus, theses stability results showed that the tablets prepared by the process described herein were both physically and chemical stable.

Moreover, the content uniformity test of theses tablets showed that the tablets were uniform and consistent in their doxycycline content both initially and after three months storage at 25°C/60% RH and at 40°C/75% RH, respectively.

The dissolution of the tablets in Bottles A and B was measured according to the standard dissolution procedures. Briefly, the dissolution tests were first conducted in 750 ml of 0.1 N HCl solution at about pH 1.1 for 120 minutes, and then in potassium phosphate buffer solution at about pH 6 by adding 200 ml of 0.1 N NaOH/200mM potassium phosphate solution to the remaining dissolution solution and adjusting the pH to about 6 with a NaOH or HCl solution. A volume of 4 ml sample was drawn at each predetermined time point shown in Tables 1 and 2. All dissolution tests were conducted at 37°C. The dissolution data for Bottles A and B are tabulated in Tables 1 and 2 below.

**Table 1: Dissolution Data for Bottle A: Storage at 25°C, 60% RH**

| D**issolution Data (% I.c.)** | **Initial** | **1 Month Storage** | **2 Months Storage** | **3 Months Storage** |
|---|---|---|---|---|
| Mean Value 30 min | 64 | 62 | 64 | 66 |
| RSD at 30 min | 6.1 | 18.5 | 4.3 | 11.1 |
| Mean Value at 60 min | 72 | 72 | 72 | 75 |
| RSD at 60 min | 8.1 | 6.9 | 7.6 | 5.3 |
| Mean value at 120 min | 72 | 74 | 72 | 77 |
| RSD at 120 min | 8.8 | 5.4 | 7.9 | 4.2 |
| Mean Value at 150 min | 95 | 95 | 95 | 96 |
| RSD at 150 min | 6.1 | 4.0 | 5.1 | 2.5 |
| Mean Value at 180 min | 97 | 100 | 98 | 99 |
| RSD at 180 min | 6.3 | 4.0 | 5.5 | 3.0 |
| Mean Value at 240 min | 97 | 100 | 98 | 101 |
| RSD at 240 min | 6.2 | 4.0 | 5.5 | 3.2 |

**Table 2: Dissolution Data for Bottle B: Storage at 40°C, 75% RH**

| **Dissolution Data (% I.c.)** | **Initial** | **1 Month Storage** | **2 Months Storage** | **3 Months Storage** |
|---|---|---|---|---|
| Mean Value 30 min | 64 | 67 | 65 | 59 |
| RSD at 30 min | 6.1 | 13.2 | 5.3 | 18.7 |
| Mean Value at 60 min | 72 | 76 | 71 | 68 |
| RSD at 60 min | 8.1 | 7.4 | 6.2 | 10.2 |
| Mean value at 120 min | 72 | 77 | 71 | 70 |
| RSD at 120 min | 8.8 | 7.3 | 6.6 | 6.8 |
| Mean Value at 150 min | 95 | 99 | 93 | 89 |
| RSD at 150 min | 6.1 | 5.1 | 5.4 | 5.8 |
| Mean Value at 180 min | 97 | 102 | 96 | 93 |
| RSD at 180 min | 6.3 | 5.8 | 5.4 | 5.6 |
| Mean Value at 240 min | 97 | 102 | 96 | 94 |
| RSD at 240 min | 6.2 | 5.8 | 5.1 | 5.7 |

The dissolution data showed the tablets prepared by the process described herein yielded comparable dissolution profiles after three month storage at 25°C/60% RH and at 40°C/75% RH compared with the initial samples. These results further confirmed that the tablets prepared by the process described herein were both physically and chemical stable.

### Example 3 Preparation of a Capsule Composition

### Preparation of Layered IR Pellets Containing Doxycycline Monohydrate

A dispersion of doxycycline monohydrate was prepared as follows: To 5.725 kilograms of deionized water were added 0.113 kilogram hydroxypropyl methylcellulose and 1.5 kilograms of doxycycline monohydrate, followed by moderate mixing, using a stirring paddle for 30 minutes. The drug dispersion was sprayed onto sugar seeds [595 µm/500 µm (30/35 mesh)] in a 22.9 cm (9") Wurster Column of a GPCG-15 fluid bed processor. Until the entire dispersion was applied, the pellets were dried in the column for 5 minutes. The drug-loaded pellets were discharged from the Wurster Column and passed through a 841 µm (20 mesh) screen. A protective coat (e.g., OPADRY® beige) also can be applied onto the IR beads to provide color or physical protection.

### Preparation of Enteric Coated Pellets Containing Doxycycline Monohydrate

The EUDRAGIT® L30D55 coating dispersion was prepared by adding 0.127 kilogram of triethyl citrate into 3.538 kilograms of EUDRAGIT® L30D55 (solid content: 1.061 kilograms) and stirring for at least 30 minutes. Talc 0.315 kilogram was dispersed into 2.939 kilograms of deionized water. The plasticized EUDRAGIT® L30D55 was combined with the talc dispersion and screened through a 250 µm (60 mesh) screen. The resulting combined dispersion was sprayed onto drug-loaded pellets (3.5 kilograms) prepared according to Example 1 in a 22.9 cm (9") Wurster Column of a GPCG-15 fluid bed processor. A protective coat (e.g., OPADRY® beige) may be applied onto the DR beads to provide color or physical protection.

### Encapsulation of Drug-Loaded Pellets and Enteric Coated Pellets

Capsules can be prepared by filling the drug-loaded pellets and enteric coated pellets individually into appropriate sized capsule shells. The ratio between the drug-loaded pellets and enteric-coated pellets is 75:25, the fill weight of drug-loaded pellets can be calculated based on the actual potency of the drug-loaded pellets to deliver 30 mg doxycycline; the fill weight of enteric-coated pellets also can be calculated based on the actual potency of the enteric-coated pellets to deliver 10 mg doxycycline. Romoco CD5 or MG-2 pellet filling machine can be used to accurately fill the pellets into the desired capsule shells.

Size 0 capsules containing a ratio of 75:25 of drug-loaded IR pellets to enteric coated DR pellets were prepared as follows. The IR and DR pellets were prepared as set forth above. From the assay value of the doxycycline used to make the pellets, it was determined that 41.26 mg potency of the capsules would correspond to an actual strength of 40 mg doxycycline. The potency of the IR pellets was 194 mg doxycycline per gram of pellets (mg/g), and for the DR pellets was 133 mg/g. Accordingly, it was calculated that for each capsule the fill weight of IR beads would be 159.5 mg, and for DR beads 77.6 mg, corresponding to 75:25 of IR:DR of a 40 mg capsule.

### Example 4 A Phase II Clinical Study

A phase 2, multi-center, randomized, double-blind, parallel group, placebo-controlled study was conducted. The treatments were (1) 40 mg doxycycline tablet containing 30 mg IR and 10 mg DR (CD2575/101); (2) doxycycline 100 mg capsules; and (3) placebos (double dummy), which were administered orally once daily.

Patients having moderate or severe acne (25 to 75 inflammatory lesions on the face (including the nose) were subjects in the study. Total 660 subjects (220/group) were divided in a 1:1:1 randomization for the three treatments. The treatments lasted 16 weeks.

The following were measured for efficacy studies: change from baseline to week 16 (LOCF, ITT) in inflammatory lesion counts (primary endpoint); success rate at week 16 (LOCF, ITT); percent change in inflammatory lesion counts from baseline to week 16 (LOCF, ITT); percent change in total lesion counts from baseline to week 16 (LOCF, ITT); change from baseline to week 16 (LOCF, ITT) in non-inflammatory lesion counts; global assessment for inflammatory lesions of truncal acne at baseline, week 12, and week 16/ET visit, etc.

The investigator evaluated the subject's acne at every study visit using the Investigator's Global Assessment (Inflammatory) scale as that described above. The IGA scale in this study focused on inflammatory lesions as the mode of study drug action in acne vulgaris is primarily anti-inflammatory.

Onset of efficacy was to be determined for the change in inflammatory lesions and for the success rate, by analyzing time points earlier than week 16.

The safety studies included standard safety reporting. Adverse events of special interest included phototoxicity; pseudotumor cerebri; and pseudomembranous colitis. Vital signs were observed by the investigator at each visit. Lab tests were conducted at 0, 8 & 16 weeks.

It was found that CD2575/101 40 mg was more effective than placebo in primary endpoint (absolute change from baseline in Inflammatory lesion count) at Week 16 and Week 12 and has a superior success rate than the placebo at week 16 but not (statistically) at Week 12. See Table 1. The study was not powered to show difference in success rate.

It was surprisingly discovered that CD2575/101 40 mg, although at a dosage level much lower than doxycycline 100 mg, resulted in same or even better efficacy, e.g., in changes in inflammatory lesion count and success rate. See Tables 1 and 2.

**Table 1. Reduction from baseline in inflammatory lesion count and success rate, ITT (LOCF) Population (p-value)**

| **Time Point, ITT** | **CD2475/101 40 mg** | **Doxycycline 100 mg** | **Placebo** |
|---|---|---|---|
| **Reduction in Intl Lesion Count** | | | |
| **Week 12 (LOCF)** | 15.6 (0.003) | 12.2 (0.604) | 11.9 |
| delta vs placebo | 3.7 | 0.3 | |
| **Week 16 (LOCF)** | 16.1 (0.006) | 12.9 (0.595) | 12.6 |
| delta vs placebo | 3.5 | 0.3 | |
| **Success Rate: n(%)** | | | |
| **Week 12 (LOCF)** | 10.2%(0.198) | 5.4%(0.536) | 6.8% |
| delta vs placebo | 3.4 | -1.4 | |
| **Week 16 (LOCF)** | 14.4% (0.025) | 13.8% (0.035) | 7.7% |
| delta vs placebo | 6.7 | 6.1 | |
| p-values refer to comparisons versus placebo. | | | |

It was further surprisingly discovered that CD2575/101 40 mg significantly reduced the non-inflammatory lesion count than doxycycline 100 mg did. See Figure 1.

In addition, CD2575/101 40mg significantly reduced the total lesion count than doxycycline 100 mg did. See Table 2.

**Table 2. Median Percent Change in Inflammatory Lesion Count and in Total Lesion Count ITT (LOCF) Population**

| **Time Point, ITT** | **CD2475/101 40 mg (1)** | **Doxycycline 100 mg (2)** | **Placebo (3)** | **(1) and (3)** | **(1) and (2)** | **(2) and (3)** |
|---|---|---|---|---|---|---|
| **Inflammatory Lesion Count** | | | | | | |
| **Week 12 (LOCF)** | | | | | | |
| Median %Change (SD) | -51.7 (29.99) | -46.7 (40.04) | -43.5 (44.25) | | | |
| P-value | | | | 0.002 | 0.173 | 0.199 |

| **Week 16 (LOCF)** | | | | | | |
|---|---|---|---|---|---|---|
| Median %Change (SD) | -51.6 (31.72) | -47.3 (40.90) | -44.3 (44.45) | | | |
| P-value | | | | 0.003 | 0.106 | 0.703 |
| | | | | | | |

| **Total Lesion Count** | | | | | | |
|---|---|---|---|---|---|---|
| **Week 12 (LOCF)** | | | | | | |
| Median %change (SD) | -42.2 (30.98) | -30.6 (46.56) | -32.5 (39.58) | | | |
| P-value | | | | < 0.001 | 0.031 | 0.344 |

| **Week 16 (LOCF)** | | | | | | |
|---|---|---|---|---|---|---|
| Median %Change (SD) | -41.7 (32.24) | -35.9 (43.94) | -34.1 (38.05) | | | |
| P-value | | | | 0.004 | 0.026 | 0.972 |

It was found that CD2575/101 40 mg had a superior safety profile compared to doxycycline 100 mg, e.g., 11 vs 38 drug related adverse effects (AEs: nausea, vomiting & headaches). See also Table 3.

**Table 3. Adverse Effects of 100mg, and 40 mg doxycycline v. placebo**

| **Preferred Tem*** | **CD2475/101 40 mg (N=216)** | **Doxycycline 100 mg (N=223)** | **Placebo (N=222)** |
|---|---|---|---|
| **Total Number of AE(s)** | **11** | **50** | **11** |
| Total Number of Subjects with AE(s) | 8 (3.7%) | 38 (17.0%) | 9 (4.1%) |
| Nausea | 3 (1.4%) | 12 (5.4%) | 2 (0.9%) |
| Dizziness | 2 (0.9%) | 1 (0.4%) | 0 |
| Abdominal discomfort | 1 (0.5%) | 4 (1.8%) | 0 |
| Headache | 1 (0.5%) | 6 (2.7%) | 0 |
| Tinnitus | 1 (0.5%) | 0 | 0 |
| Vulvovaginal mycotic infection | 1 (0.5%) | 1 (0.4%) | 0 |
| Urticaria | 0 | 2 (0.9%) | 0 |
| Vomiting | 0 | 14 (6.3%) | 1 (0.5%) |

## Claims

1. A pharmaceutical composition comprising 40 mg doxycycline or a pharmaceutically acceptable salt thereof for use in treating acne in a subject in need of the treatment, wherein the pharmaceutical composition comprises 30 mg doxycycline in an immediate release portion and 10 mg doxycycline in a delayed-release portion and wherein the pharmaceutical composition is to be orally administered to the subject once daily for a treatment period of 16 weeks.

2. The pharmaceutical composition for use according to claim 1, wherein the pharmaceutical composition is in a tablet form.

3. The pharmaceutical composition for use according to claim 1, wherein the pharmaceutical composition is in a capsule form.

4. The pharmaceutical composition for use according to claim 1, wherein the said treatment with said pharmaceutical achieves the same or better efficacy than treatment with 100 mg doxycycline or the pharmaceutically acceptable salt thereof.

5. The pharmaceutical composition for use according to claim 1, wherein the said treatment reduces the inflammatory lesion counts of the subject.

6. The pharmaceutical composition for use according to claim 1, wherein the said treatment reduces the non-inflammatory lesion counts of the subject.

7. The pharmaceutical composition for use according to claim 1, wherein the said treatment increases the success rate.

## Patentansprüche

1. Pharmazeutische Zusammensetzung, umfassend 40 mg Doxycyclin oder ein pharmazeutisch verträgliches Salz davon zur Verwendung bei der Behandlung von Akne in einem Patienten, der der Behandlung bedarf, wobei die pharmazeutische Zusammensetzung 30 mg Doxycyclin in einer sofortigen Freisetzungsportion und 10 mg Doxycyclin in einer verzögerten Freisetzungsportion umfasst, und wobei die pharmazeutische Zusammensetzung an den Patienten einmal täglich für einen Behandlungszeitraum von 16 Wochen oral verabreicht wird

2. Pharmazeutische Zusammensetzung zur Verwendung nach Anspruch 1, wobei die pharmazeutische Zusammensetzung in einer Tablettenform vorliegt.

3. Pharmazeutische Zusammensetzung zur Verwendung nach Anspruch 1, wobei die pharmazeutische Zusammensetzung in einer Kapselform vorliegt.

4. Pharmazeutische Zusammensetzung zur Verwendung nach Anspruch 1, wobei die Behandlung mit dem Pharmazeutikum die gleiche oder bessere Wirksamkeit als Behandlung mit 100 mg Doxycyclin oder einem pharmazeutisch verträglichen Salz davon erreicht.

5. Pharmazeutische Zusammensetzung zur Verwendung nach Anspruch 1, wobei die Behandlung die Anzahl inflammatorischer Läsionen des Patienten verringert.

6. Pharmazeutische Zusammensetzung zur Verwendung nach Anspruch 1, wobei die Behandlung die Anzahl nicht-inflammatorischer Läsionen des Patienten verringert.

7. Pharmazeutische Zusammensetzung zur Verwendung nach Anspruch 1, wobei die Behandlung die Erfolgsrate erhöht.

## Revendications

1. Une composition pharmaceutique comprenant 40 mg de doxycycline ou un sel pharmaceutiquement acceptable en dérivant pour une utilisation dans le traitement de l'acné d'un sujet nécessitant un tel traitement, dans laquelle la composition pharmaceutique comprend 30 mg de doxycycline dans une fraction à libération immédiate et 10 mg de doxycycline dans une fraction à libération retardée et dans laquelle la composition pharmaceutique est destinée à être administrée par voie orale audit sujet une fois par jour pour une période de traitement de 16 semaines.

2. La composition pharmaceutique pour une utilisation selon la revendication 1, dans laquelle la composition pharmaceutique est sous forme de comprimés.

3. La composition pharmaceutique pour une utilisation selon la revendication 1, dans laquelle la composition pharmaceutique est sous forme de capsule.

4. La composition pharmaceutique pour une utilisation selon la revendication 1, dans laquelle ledit traitement avec ledit médicament donne lieu à une efficacité semblable ou meilleure que le traitement par 100 mg de doxycycline ou par le sel pharmaceutiquement acceptable en dérivant.

5. La composition pharmaceutique pour une utilisation selon la revendication 1, dans laquelle ledit traitement réduit le nombre de lésions inflammatoires du sujet.

6. La composition pharmaceutique pour une utilisation selon la revendication 1, dans laquelle ledit traitement réduit le nombre de lésions non-inflammatoires du sujet.

7. La composition pharmaceutique pour une utilisation selon la revendication 1, dans laquelle ledit traitement augmente le taux de succès.
